(19) Europäisches Patentamt / European Patent Office / Office européen des brevets

(11) **EP 3 797 426 B1**

(12) **FASCICULE DE BREVET EUROPEEN**

(45) Date de publication et mention
de la délivrance du brevet:
**10.04.2024   Bulletin 2024/15**

(21) Numéro de dépôt: **19728497.9**

(22) Date de dépôt: **03.05.2019**

(51) Classification Internationale des Brevets (IPC):
*G16H 50/50* *(2018.01)*   *G16H 20/17* *(2018.01)*
*A61B 5/00* *(2006.01)*   *A61B 5/145* *(2006.01)*
*A61B 5/11* *(2006.01)*   *A61B 5/024* *(2006.01)*

(52) Classification Coopérative des Brevets (CPC):
**A61B 5/4839; A61B 5/1118; A61B 5/14532;
A61B 5/7275; G16H 20/17; G16H 50/50;**
A61B 5/024; A61B 5/4866; A61B 2562/0219

(86) Numéro de dépôt international:
**PCT/FR2019/051026**

(87) Numéro de publication internationale:
**WO 2019/224447 (28.11.2019 Gazette 2019/48)**

(54) **SYSTEME AUTOMATISE DE CONTROLE DE LA GLYCEMIE D'UN PATIENT**

AUTOMATISIERTES SYSTEM ZUR ÜBERWACHUNG DES BLUTZUCKERS EINES PATIENTEN

AUTOMATED SYSTEM FOR MONITORING A PATIENT'S BLOOD SUGAR

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **22.05.2018   FR 1800493**

(43) Date de publication de la demande:
**31.03.2021   Bulletin 2021/13**

(73) Titulaire: **Commissariat à l'Energie Atomique et
aux Energies
Alternatives
75015 Paris (FR)**

(72) Inventeurs:
• **BLANC, Romain**
  **38000 Grenoble (FR)**
• **DORON, Eléonore-Maeva**
  **38054 Grenoble Cedex 09 (FR)**
• **ROMERO UGALDE, Hector-Manuel**
  **38000 Grenoble (FR)**

(74) Mandataire: **Cabinet Beaumont
4, Place Robert Schuman
B.P. 1529
38025 Grenoble Cedex 1 (FR)**

(56) Documents cités:
**WO-A1-2018/007161     US-A1- 2010 057 043
US-A1- 2015 217 052**

• **PETER G JACOBS ET AL: "Development of a fully
automated closed loop artificial pancreas control
system with dual pump delivery of insulin and
glucagon", ENGINEERING IN MEDICINE AND
BIOLOGY SOCIETY,EMBC, 2011 ANNUAL
INTERNATIONAL CONFERENCE OF THE IEEE,
IEEE, 30 août 2011 (2011-08-30), pages 397-400,
XP032318727, DOI: 10.1109/IEMBS.2011.6090127
ISBN: 978-1-4244-4121-1**
• **UTZ TILMAN ET AL: "Model of the glucose-insulin
system of type-1 diabetics and
optimization-based bolus calculation", 2014
UKACC INTERNATIONAL CONFERENCE ON
CONTROL (CONTROL), IEEE, 9 juillet 2014
(2014-07-09), pages 579-584, XP032654591, DOI:
10.1109/CONTROL.2014.6915204**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente demande de brevet revendique la priorité de la demande de brevet français FR18/00493 qui sera considérée comme faisant partie intégrante de la présente description.

### Domaine

La présente demande concerne le domaine des systèmes automatisés de contrôle de glycémie, et vise plus particulièrement, dans un tel système, la détermination d'un coefficient représentatif de la sensibilité à l'insuline du patient.

### Exposé de l'art antérieur

On a déjà proposé, par exemple dans la demande de brevet français N°1658881 (B15018/DD16959) déposée le 21 septembre 2016, dans la demande de brevet français N°1658882 (B15267/ DD17175) déposée le 21 septembre 2016, et dans la demande de brevet français N°1756960 (B15860/DD18588) déposée le 21 juillet 2017, des systèmes automatisés de régulation de glycémie, aussi appelés pancréas artificiels, permettant de réguler automatiquement les apports en insuline d'un patient diabétique à partir de son historique de glycémie, de son historique de prise de repas, et de son historique d'injection d'insuline.

Les systèmes de régulation décrits dans les demandes de brevet susmentionnées sont des systèmes de type MPC (de l'anglais "Model-based Prédictive Control"), aussi appelés systèmes à commande prédictive, dans lesquels la régulation de la dose d'insuline administrée tient compte d'une prédiction de l'évolution future de la glycémie du patient, réalisée à partir d'un modèle physiologique décrivant l'assimilation de l'insuline par le corps du patient et son impact sur la glycémie du patient.

Plus généralement, de nombreux systèmes automatisés de contrôle de glycémie tiennent compte de l'historique de glycémie, de l'historique de prise de repas, et de l'historique d'injection d'insuline d'un patient pour déterminer des doses d'insuline à administrer au patient en vue de maintenir sa glycémie dans une plage souhaitée.

Dans les systèmes automatisés de contrôle de glycémie, un paramètre qui joue un rôle essentiel dans la détermination des doses d'insuline à administrer au patient est le coefficient de sensibilité à l'insuline du patient, aussi appelée coefficient de sensibilité de compensation, ou ratio de compensation, c'est-à-dire la quantité d'insuline nécessaire pour faire baisser la glycémie de un gramme par litre (en UI/g/l - où UI désigne une unité internationale d'insuline, soit l'équivalent biologique d'environ 0,0347 mg d'insuline humaine). Tels systèmes sont connus des documents US 2010/057043 A1 et US 2015/217052 A1.

Un problème qui se pose est que le coefficient de sensibilité à l'insuline peut varier de façon significative d'un patient à un autre, ou, chez un même patient, en fonction des conditions dans lesquelles se trouve le patient, et notamment en fonction de l'activité physique du patient.

En pratique, les systèmes automatisés de contrôle de glycémie connus se basent sur un coefficient de sensibilité à l'insuline fixe, par exemple fourni par le diabétologue du patient. Il n'est donc pas tenu compte des variations à court terme du coefficient. Il en résulte que les quantités d'insuline injectées au patient sont parfois inadaptées, entraînant un risque d'hyperglycémie ou d'hypoglycémie.

### Résumé

Ainsi, invention prévoit un système selon la revendication 1.

Selon un mode de réalisation, le système comporte en outre un dispositif d'injection d'insuline, et l'unité de traitement et de contrôle est configurée pour commander le dispositif d'injection d'insuline en tenant compte du coefficient CR.

Selon un mode de réalisation, l'unité de traitement et de contrôle est configurée pour prédire, à partir d'un deuxième modèle mathématique, l'évolution future de la glycémie du patient sur une période de prédiction, et à commander le dispositif d'injection d'insuline en tenant compte de cette prédiction.

Selon un mode de réalisation, le premier modèle mathématique est une fonction d'équation

$$CR = f_{CR}(IPA, G^r) = a \times IPA^b + c \times G^{r^d} + e$$

où a, b, c, d et e sont des paramètres spécifiques au patient.

Selon un mode de réalisation, le premier modèle mathématique est une fonction d'équation

$$CR = G^r \times f_{CR}(IPA) = G^r \times ( a \times IPA^b + c)$$

où a, b et c sont des paramètres spécifiques au patient.

Selon un mode de réalisation, l'unité de traitement et de contrôle est configurée pour mettre en oeuvre une étape de calibration automatique du premier modèle $f_{CR}$ en tenant compte d'un historique de glycémie mesurée par le capteur, d'un historique d'insuline injectée au patient, d'un historique d'ingestion de glucose par le patient, et d'un historique du signal d'activité physique PA du patient, sur une période d'observation passée.

Selon un mode de réalisation, chaque événement de mesure correspond à une plage temporelle continue allant d'un instant initial $t_{init}$ à un instant final $t_{final}$, répondant aux critères suivants :

- l'instant $t_{init}$ se situe dans une phase d'hyperglycémie, c'est-à-dire une phase dans laquelle la glycémie du patient est supérieure à un seuil prédéterminé ;
- un bolus de correction, c'est-à-dire une dose d'insuline a été administré au patient après le début de la phase d'hyperglycémie et avant l'instant $t_{init}$, en vue de limiter la durée de la phase d'hyperglycémie ;
- la glycémie du patient décroit de façon continue entre l'instant initial $t_{init}$ et l'instant final $t_{final}$ ; et
- aucune ingestion de glucose par le patient n'a été effectuée entre l'instant $t_{init}$ - $T_j$ et l'instant $t_{final}$, où $T_j$ est une durée de jeun prédéterminée.

Selon un mode de réalisation, l'unité de traitement et de contrôle est configurée pour, lors de l'étape de calibration automatique, déterminer le premier modèle mathématique $f_{CR}$ par régression à partir de ladite pluralité de valeurs du coefficient de sensibilité à l'insuline réel $CR^r$.

Selon un mode de réalisation, la fonction H est une fonction exponentielle décroissante.

Selon un mode de réalisation, le dispositif de mesure comprend une interface utilisateur par laquelle le patient déclare les activités physiques qu'il réalise.

Selon un mode de réalisation, le dispositif de mesure comprend un ou plusieurs capteurs adaptés à mesurer des grandeurs représentatives de l'activité physique du patient.

Selon un mode de réalisation, le dispositif de mesure comprend un capteur de mouvement et/ou un capteur de rythme cardiaque.

Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

la figure 1 représente de façon schématique, sous forme de blocs, un exemple d'un système automatisé de régulation de la glycémie d'un patient selon un mode de réalisation ;
la figure 2 est une représentation simplifiée d'un modèle physiologique utilisé dans le système de la figure 1 pour prédire l'évolution future de la glycémie du patient ;
la figure 3 est un diagramme illustrant un exemple d'un procédé automatisé de régulation de glycémie pouvant être mis en oeuvre par le système de la figure 1 ;
les figures 4A et 4B sont des diagrammes représentant l'évolution, en fonction du temps, respectivement d'un signal PA représentatif de l'activité physique du patient et d'un signal IPA représentatif de l'influence de l'activité physique du patient sur son coefficient de sensibilité à l'insuline ;
les figures 5A et 5B sont des diagrammes représentant l'évolution du coefficient de sensibilité à l'insuline d'un patient en fonction de sa glycémie et du signal IPA ;
la figure 6 est une représentation simplifiée d'un modèle mathématique utilisé dans le système de la figure 1 pour déterminer le coefficient de sensibilité à l'insuline du patient ; et
la figure 7 est un diagramme illustrant un exemple d'un procédé pouvant être mis en oeuvre par le système de la figure 1 pour mettre à jour le modèle mathématique de la figure 6.

Description détaillée

De mêmes éléments ont été désignés par de mêmes références dans les différentes figures et, de plus, les diverses figures ne sont pas tracées à l'échelle. Par souci de clarté, seuls les éléments utiles à la compréhension des modes de réalisation décrits ont été représentés et sont détaillés. En particulier, la réalisation matérielle de l'unité de traitement et de contrôle des systèmes décrits n'a pas été détaillée, la réalisation d'une telle unité de traitement et de contrôle étant à la portée de l'homme du métier à partir des indications fonctionnelles de la présente description. En outre, le dispositif de mesure de glycémie et le dispositif d'injection d'insuline des systèmes décrits n'ont pas été détaillés, les modes de réalisation décrits étant compatibles avec tous ou la plupart des dispositifs de mesure de glycémie et d'injection d'insuline connus. Sauf précision contraire, les expressions "approximativement", "sensiblement", et "de l'ordre de" signifient à 10 % près, de préférence à 5 % près.

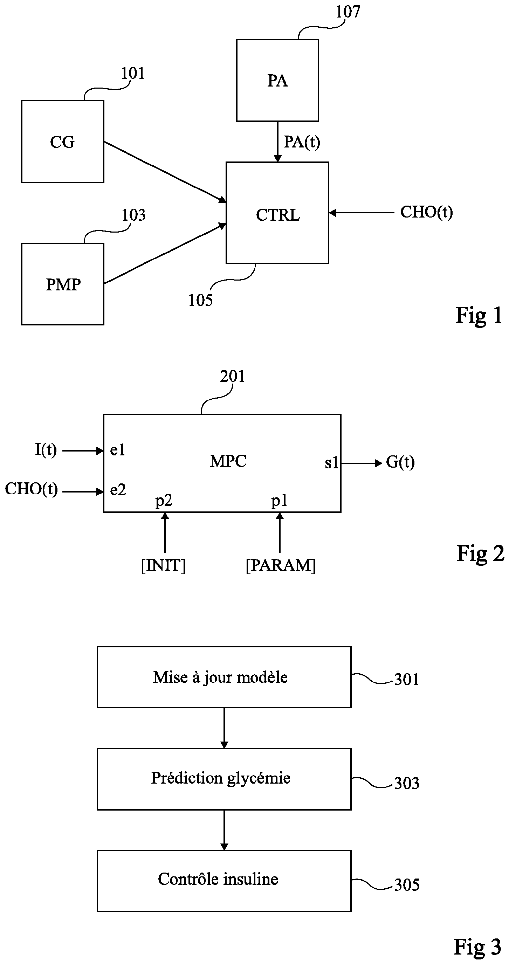

La figure 1 représente de façon schématique, sous forme de blocs, un exemple d'un mode de réalisation d'un système automatisé de régulation de la glycémie d'un patient.

Le système de la figure 1 comprend un capteur 101 (CG) adapté à mesurer la glycémie du patient. En fonctionnement normal, le capteur 101 peut être positionné à demeure sur ou dans le corps du patient, par exemple à hauteur de son abdomen. Le capteur 101 est par exemple un capteur de type CGM (de l'anglais "Continuous Glucose Monitoring" - surveillance continue de glycémie), c'est-à-dire un capteur adapté à mesurer en continu, par exemple de façon périodique (par exemple au moins une fois toutes les cinq minutes) la glycémie du patient. Le capteur 101 est par exemple un capteur de glycémie en sous-cutané.

Le système de la figure 1 comprend en outre un dispositif d'injection d'insuline 103 (PMP), par exemple un dispositif d'injection en sous-cutané. Le dispositif 103 est par exemple un dispositif d'injection automatique de type pompe à insuline, comportant un réservoir d'insuline relié à une aiguille d'injection implantée sous la peau du patient, la pompe pouvant être commandée électriquement pour injecter automatiquement des doses d'insuline déterminées à des instants déterminés. En fonctionnement normal, le dispositif d'injection 103 peut être positionné à demeure dans ou sur le corps du patient, par exemple au niveau de son abdomen.

Le système de la figure 1 comprend en outre une unité de traitement et de contrôle 105 (CTRL) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie du patient mesurées par le capteur 101, et à commander électriquement le dispositif 103 pour injecter au patient des doses d'insuline déterminées à des instants déterminés. Dans cet exemple, l'unité de traitement et de contrôle 105 est en outre adaptée à recevoir, par l'intermédiaire d'une interface utilisateur non détaillée, des données CHO(t) représentatives de l'évolution, en fonction du temps, de la quantité de glucose ingérée par le patient.

L'unité de traitement et de contrôle 105 est adaptée à déterminer les doses d'insuline à injecter au patient en tenant compte notamment de l'historique de glycémie mesurée par le capteur 101, de l'historique d'insuline injectée par le dispositif 103, et de l'historique d'ingestion de glucose par le patient. Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile porté par le patient tout au long de la journée et/ou de la nuit, par exemple un dispositif de type smartphone configuré pour mettre en oeuvre un procédé de régulation du type décrit ci-après.

Dans l'exemple de la figure 1, l'unité de traitement et de contrôle 105 est adaptée à déterminer la quantité d'insuline à administrer au patient en tenant compte d'une prédiction de l'évolution future de sa glycémie en fonction du temps. Plus particulièrement, l'unité de traitement et de contrôle 105 est adaptée, à partir de l'historique d'insuline injectée et de l'historique de glucose ingéré, et en se basant sur un modèle mathématique, par exemple un modèle physiologique décrivant l'assimilation de l'insuline par le corps du patient et son impact sur la glycémie, à déterminer une courbe représentative de l'évolution attendue de la glycémie du patient en fonction du temps, sur une période à venir appelée période de prédiction ou horizon de prédiction, par exemple une période de 1 à 10 heures. En tenant compte de cette courbe et d'un coefficient représentatif de la sensibilité à l'insuline du patient, l'unité de traitement et de contrôle 105 détermine les doses d'insuline qu'il convient d'injecter au patient pendant la période de prédiction à venir, pour que la glycémie réelle (par opposition à la glycémie estimée à partir du modèle mathématique) du patient reste dans des limites acceptables, et en particulier pour limiter les risques d'hyperglycémie ou d'hypoglycémie.

La figure 2 est une représentation simplifiée d'un modèle mathématique 201 (MPC) utilisé dans le système de la figure 1 pour prédire l'évolution future de la glycémie du patient. Sur la figure 2, le modèle est représenté sous la forme d'un bloc de traitement comportant :

une entrée e1 sur laquelle est appliquée un signal I(t) représentatif de l'évolution, en fonction du temps t, de la quantité d'insuline injectée au patient ;
une entrée e2 sur laquelle est appliquée un signal CHO(t) représentatif de l'évolution, en fonction du temps t, de la quantité de glucose ingérée par le patient ; et
une sortie s1 fournissant un signal G(t) représentatif de l'évolution, en fonction du temps t, de la glycémie estimée du patient.

Le modèle mathématique 201 est par exemple un modèle physiologique. A titre d'exemple, le modèle 201 est un modèle physiologique compartimental comportant, outre les variables d'entrée I(t) et CHO(t) et la variable de sortie G(t), une pluralité de variables d'état correspondant à des variables physiologiques du patient, évoluant en fonction du temps. L'évolution temporelle des variables d'état et de la variable de sortie G(t) est régie par un système d'équations différentielles comportant une pluralité de paramètres représentés sur la figure 2 par un vecteur [PARAM] appliqué sur une entrée p1 du bloc 201. La réponse du modèle physiologique est en outre conditionnée par les états initiaux ou valeurs initiales affectés aux variables d'état, représentés sur la figure 2 par un vecteur [INIT] appliqué sur une entrée p2 du bloc 201.

A titre d'exemple, le modèle physiologique 201 utilisé dans le système de la figure 1 est le modèle dit de Hovorka, décrit dans l'article intitulé "Nonlinear model prédictive control of glucose concentration in subjects with type 1 diabètes" de Roman Hovorka et al. (Physiol Meas. 2004;25:905-920), et dans l'article intitulé "Partitioning glucose distribution/transport, disposal, and endogenous production during IVGTT", de Roman Hovorka et al. (Am J Physiol Endocrinol Metab 282: E992-E1007, 2002). Plus généralement, tout autre modèle physiologique décrivant l'assimilation de l'insuline par le corps d'un patient et son effet sur la glycémie du patient peut être utilisé.

Parmi les paramètres du vecteur [PARAM], certains peuvent être considérés comme constants pour un patient donné. D'autres paramètres, appelés ci-après paramètres temps-dépendants, sont en revanche susceptibles d'évoluer dans le temps. Du fait de cette variabilité de certains paramètres du système, il est en pratique nécessaire de ré-étalonner ou re-calibrer régulièrement le modèle en cours d'utilisation, par exemple toutes les 1 à 20 minutes, par exemple toutes les 5 minutes, pour s'assurer que les prédictions du modèle restent pertinentes. Cette mise à jour du modèle, aussi appelée personnalisation du modèle, doit de préférence pouvoir être réalisée de façon automatique par le système de la figure 1, c'est-à-dire sans qu'il soit nécessaire de mesurer physiquement les paramètres temps-dépendants du système sur le patient puis de les transmettre à l'unité de traitement et de contrôle 105.

La figure 3 est un diagramme illustrant un exemple d'un procédé automatisé de régulation de glycémie pouvant être mis en oeuvre par le système de la figure 1.

Ce procédé comprend une étape 301 de re-calibration ou mise à jour du modèle, pouvant par exemple être répétée à intervalles réguliers, par exemple toutes les 1 à 20 minutes. Lors de cette étape, l'unité de traitement et de contrôle 105 met en oeuvre un procédé de ré-estimation des paramètres temps-dépendants du modèle en tenant compte des données d'insuline effectivement injectée par le dispositif 103 et des données de glycémie réelle mesurée par le capteur 101 pendant une période d'observation passée de durée $\Delta T$, par exemple une période de 1 à 10 heures précédant l'étape de calibration. Plus particulièrement, lors de l'étape de calibration, l'unité de traitement et de contrôle 105 simule le comportement du patient sur la période d'observation passée à partir du modèle physiologique (en tenant compte des éventuelles ingestions de glucose et injections d'insuline pendant cette période), et compare la courbe de glycémie estimée par le modèle à la courbe de glycémie réelle mesurée par le capteur pendant cette même période. L'unité de traitement et de contrôle 105 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation. A titre d'exemple, l'unité de traitement et de contrôle recherche un jeu de paramètres conduisant à minimiser un indicateur m représentatif de l'aire entre la courbe de glycémie estimée par le modèle et la courbe de glycémie réelle pendant la période d'observation, aussi appelé écart quadratique moyen entre la glycémie estimée et la glycémie réelle, par exemple défini comme suit :

$$ m = \frac{1}{\Delta T} \sum_{t=t_0-\Delta T}^{t_0} |G^r(t) - G(t)|^2 $$

où t est une variable temps discrétisée, $t_0-\Delta T$ correspond à l'instant de début de la phase d'observation passé, $t_0$ correspond à l'instant de fin de la phase d'observation passée (correspondant par exemple à l'instant de début de l'étape de calibration du modèle), $G^r$ est la courbe d'évolution temporelle de la glycémie réelle mesurée par le capteur 101 pendant la période $[t_0-\Delta T, t_0]$, et G est la courbe de glycémie estimée à partir du modèle pendant la période $[t_0-\Delta T, t_0]$. A titre de variante, pour le calcul de l'écart quadratique moyen, la variable $\Delta T$ peut être remplacée par le nombre de mesures réalisées pendant la période d'observation passée. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût.

On notera que lors de l'étape 301, outre les paramètres temps-dépendants du modèle, l'unité de traitement et de contrôle 105 définit un vecteur [INIT] d'états initiaux (états à l'instant $t_0-\Delta T$) des variables d'état du modèle, pour pouvoir simuler le comportement du patient à partir du modèle. Pour définir les états initiaux des variables d'état du modèle, une première possibilité consiste à faire l'hypothèse que, dans la période précédant la période d'observation $[t_0-\Delta T, t_0]$ sur laquelle est basée la calibration du modèle, le patient se trouvait dans un état stationnaire, avec un débit d'insuline injectée constant, et une prise alimentaire de glucose nulle. Sous cette hypothèse, toutes les dérivées du système d'équations différentielles peuvent être considérées comme nulles à l'instant initial $t_0-\Delta T$. Les valeurs à l'instant

$t_0$-$\Delta T$ des variables d'état du système peuvent alors être calculées analytiquement. Pour améliorer l'initialisation, une autre possibilité consiste à faire les mêmes hypothèses que précédemment, mais en ajoutant la contrainte que la glycémie estimée à l'instant $t_0$-$\Delta T$ soit égale à la glycémie réelle mesurée par le capteur. Pour améliorer encore l'initialisation, une autre possibilité est de considérer les états initiaux des variables d'état du modèle comme des variables aléatoires, au même titre que les paramètres temps-dépendants du modèle. Les états initiaux des variables d'état sont alors déterminés de la même façon que les paramètres temps-dépendants du modèle, c'est-à-dire que l'unité de traitement et de contrôle 105 recherche un jeu de valeurs d'états initiaux [INIT] conduisant à minimiser une grandeur représentative de l'erreur entre la courbe de glycémie estimée par le modèle et la courbe de glycémie réelle pendant la période d'observation passée.

Le procédé de la figure 3 comprend en outre, après l'étape 301, une étape 303 de prédiction, par l'unité de traitement et de contrôle 105, de l'évolution temporelle de la glycémie du patient sur une période de prédiction à venir $[t_0, t_0+T_{pred}]$ de durée $T_{pred}$, par exemple comprise entre 1 et 10 heures, à partir du modèle physiologique mis à jour à l'étape 301 et en tenant compte de l'historique d'insuline injectée au patient et de l'historique de glucose ingéré par le patient.

Le procédé de la figure 3 comprend de plus, après l'étape 303, une étape 305 de détermination, par l'unité de traitement et de contrôle 105, en tenant compte de la courbe de glycémie future prédite à l'étape 303, des doses d'insuline à injecter au patient pendant la période de prédiction à venir $[t_0, t_0+T_{pred}]$. A l'issue de cette étape, l'unité de traitement et de contrôle 105 peut programmer le dispositif d'injection 103 pour administrer les doses déterminées pendant la période de prédiction $[t_0, t_0+T_{pred}]$.

Les étapes 303 de prédiction de la glycémie et 305 de détermination des doses futures d'insuline à administrer peuvent par exemple être réitérées à chaque mise à jour du modèle physiologique (c'est-à-dire après chaque itération de l'étape 301), à chaque nouvelle ingestion de glucose signalée par le patient, et/ou à chaque nouvelle administration d'une dose d'insuline par le dispositif d'injection 103.

Selon un aspect d'un mode de réalisation, lors de l'étape 305, l'unité de traitement et de contrôle 105 estime le coefficient de sensibilité à l'insuline CR du patient en tenant compte de l'activité physique du patient. Pour cela, comme cela apparaît sur la figure 1, le système comprend un dispositif 107 de mesure d'une activité physique du patient. Le dispositif 107 est relié à l'unité de traitement et de contrôle 105, par exemple par liaison filaire ou par liaison radio (sans fil), et communique à l'unité de traitement et de contrôle 105 un signal PA(t) représentatif de l'évolution, en fonction du temps t, d'une activité physique du patient. Le dispositif 107 est par exemple un dispositif mobile porté par le patient tout au long de la journée et/ou de la nuit.

A titre d'exemple, le dispositif 107 est une simple interface utilisateur (non détaillée) par laquelle le patient déclare les activités physiques qu'il réalise. A titre d'exemple, le signal PA(t) correspond à un niveau d'intensité physique déclaré par le patient par le biais du dispositif 107. Le dispositif 107 est par exemple muni d'un clavier permettant à l'utilisateur de saisir, sur une échelle de 0 à N, où N est un entier strictement positif, par exemple égal à 3, le niveau d'intensité de l'activité physique qu'il est en train de réaliser, la valeur 0 correspondant à une activité physique considérée comme nulle ou négligeable, et la valeur N correspondant au niveau d'intensité d'activité physique maximal du patient.

A titre de variante, le dispositif 107 comprend un ou plusieurs capteurs adaptés à mesurer des grandeurs représentatives de l'activité physique du patient. A titre d'exemple, le dispositif 107 comprend au moins un capteur de mouvement (non détaillé sur la figure 1), par exemple un accéléromètre. Le dispositif 107 peut en outre comprendre un capteur du rythme cardiaque du patient (non détaillé sur la figure 1). Dans ce cas, le signal PA(t) est par exemple un signal représentatif de la dépense énergétique du patient, calculé à partir des données de sortie du ou des capteurs du dispositif 107, par exemple tel que décrit dans l'article intitulé "Prior automatic posture and activity identification improves physical activity energy expenditure prédiction from hip-worn triaxial accelerometry" de M. Garnotel et al. (Journal of Applied Physiology (1985) . 2017 Nov 30), ou dans l'article intitulé "An original piecewise model for computing energy expenditure from accelerometer and heart rate signals" de H. Romero-Ugalde et al. (Physiological measurement, 2017 Jul 28;38(8) :1599-1615).

A titre de variante, le signal PA(t) peut être une combinaison d'un signal mesuré au moyen d'un ou plusieurs capteurs du dispositif 107, et d'un signal d'intensité d'activité physique déclaré par le patient au moyen d'une interface utilisateur du dispositif 107.

Selon un aspect d'un mode de réalisation, l'unité de traitement et de contrôle 105 est configurée pour calculer, à partir du signal PA(t) représentatif de l'évolution, en fonction du temps t, de l'activité physique du patient, un signal IPA(t) représentatif de l'évolution, en fonction du temps t, de l'influence de l'activité physique passée ou en cours sur le coefficient de sensibilité à l'insuline du patient. Plus particulièrement, le signal IPA(t) est généré par convolution du signal PA(t) avec une fonction de décroissance H(t). La fonction H(t) est par exemple une fonction exponentielle décroissante. Plus généralement, toute autre fonction décroissante représentative de la diminution de l'influence d'une activité physique sur le coefficient de sensibilité à l'insuline du patient peut être utilisée, par exemple une fonction linéaire décroissante, une phase de décroissance d'une fonction parabolique ou hyperbolique, etc. La fonction de décroissance H(t) peut prendre en compte l'intensité et la durée de l'activité physique, ainsi qu'un temps caractéristique d'extinction $T_{ext}$, c'est-

à-dire une durée au-delà de laquelle l'influence de l'activité physique passée sur le coefficient de sensibilité à l'insuline est considérée comme nulle ou négligeable.

A titre d'exemple, la fonction IPA(t) est définie comme suit :

$$IPA(t) = (PA * H)(t)$$

avec :

$$H(t) = \frac{1}{\tau} e^{-\frac{t}{\tau}}$$

où $\tau$ est une variable, en min$^{-1}$, fonction de la durée et de l'intensité des activités physiques passées.

En pratique, le signal PA(t) et la fonction H(t) peuvent être échantillonnés à une période d'échantillonnage T, par exemple de l'ordre de 1 minute. La variable $\tau$ peut être calculée à chaque instant t, sur une fenêtre temporelle glissante [t-$T_{ext}$ ; t] . A titre d'exemple, la variable $\tau$ est définie comme suit :

$$\tau = \sum_{k=1}^{Next} I(k) \times (1 - \frac{k}{N_{ext}})$$

$N_{ext}$ étant un entier supérieur à 1 définissant la durée de la période d'extinction $T_{ext}$, telle que $T_{ext} = N_{ext} \times T$, et I étant un vecteur de $N_{ext}$ valeurs représentant l'intensité de l'activité physique effectuée par le patient sur la période [t-$T_{ext}$ ; t], échantillonnée à la période d'échantillonnage T. A titre d'exemple, la période d'extinction $T_{ext}$ est comprise entre 12 et 72 heures, par exemple de l'ordre de 48 heures, soit $N_{ext}$ = 2880 pour T = 1 minute.

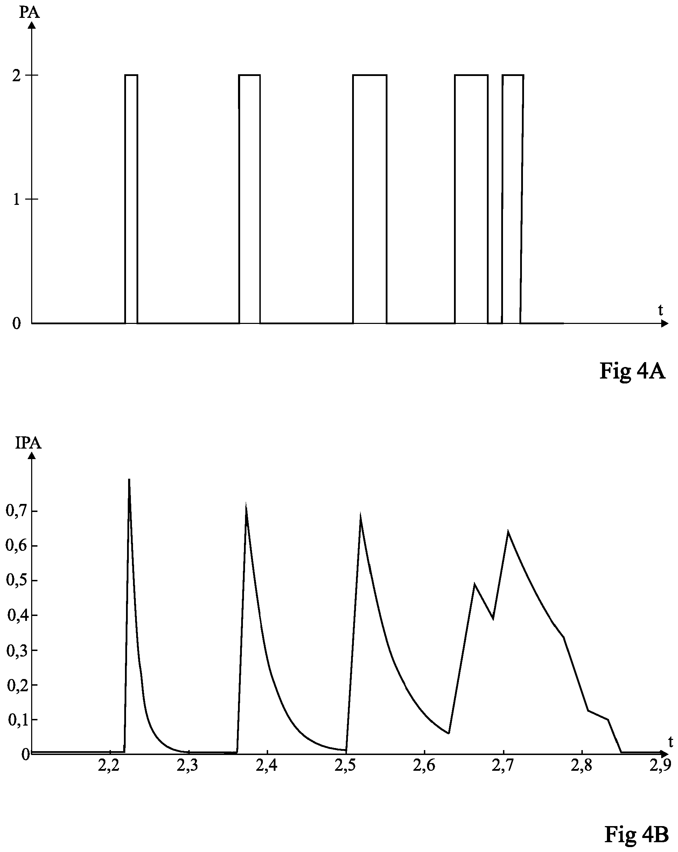

Les figures 4A et 4B sont des diagrammes représentant, à titre d'exemple illustratif, l'évolution, en fonction du temps, respectivement du signal PA (figure 4A) représentatif de l'intensité d'une activité physique effectuée par le patient, et du signal IPA (figure 4B) représentatif de l'influence de l'activité physique du patient sur son coefficient de sensibilité à l'insuline. Dans cet exemple, le signal IPA est calculé par l'unité de traitement et de contrôle à partir de la fonction de décroissance exponentielle H définie ci-dessus.

Selon un aspect d'un mode de réalisation, lors de l'étape 305 du procédé de la figure 3, l'unité de traitement et de contrôle 105 estime le coefficient de sensibilité à l'insuline CR du patient à partir d'une unique valeur du signal IPA et d'une unique valeur de glycémie mesurée par le capteur 101, en se basant sur un modèle mathématique prédéterminé. Plus particulièrement, lors de l'étape 305, l'unité de traitement et de contrôle 105 calcule le coefficient de sensibilité à l'insuline CR estimé du patient à partir d'une fonction mathématique prédéterminée $f_{CR}$ telle que $CR = G^r(t) \times f_{CR}(IPA(t))$, où $G^r$ (t) est la valeur de glycémie réelle du patient mesurée par le capteur 101 à un instant t courant, par exemple à l'instant t=$t_0$ de début de la période de prédiction considérée à l'étape 303, IPA(t) étant la valeur du signal IPA à l'instant t. Lors de l'étape 305, l'unité de traitement et de contrôle 105 détermine alors les doses futures d'insuline à administrer au patient en tenant compte du coefficient de sensibilité CR ainsi calculé.

Les inventeurs ont montré qu'il existe, pour un patient donné, une corrélation forte entre l'évolution temporelle du signal IPA du patient, et l'évolution temporelle du coefficient de sensibilité à l'insuline du patient, normalisé par rapport à la glycémie du patient. Les inventeurs ont notamment montré que l'ajustement, en temps réel, du coefficient de sensibilité à l'insuline du patient en fonction de sa glycémie instantanée et du signal IPA, permet de déterminer avec une meilleure précision les doses d'insuline futures à administrer au patient et ainsi limiter les risques d'hyperglycémie ou d'hypoglycémie.

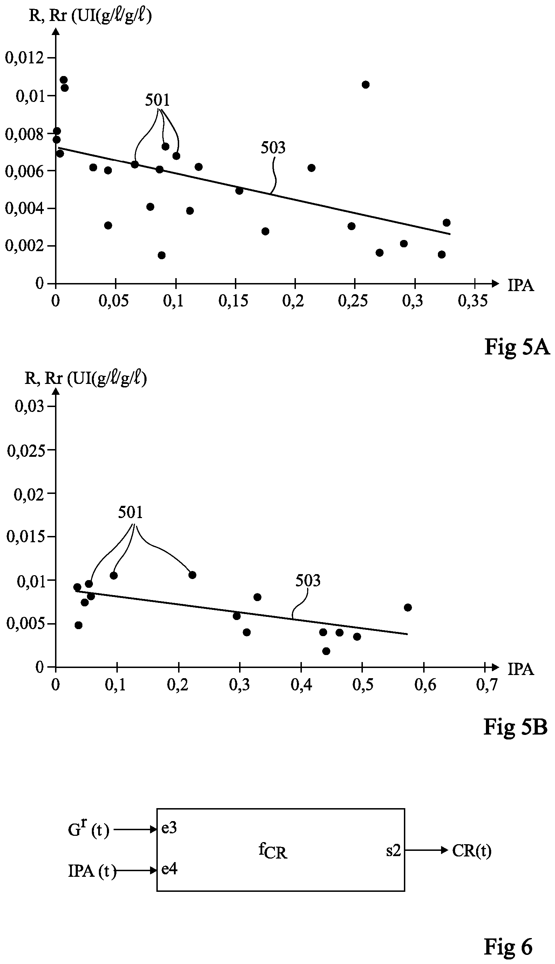

Les figures 5A et 5B sont des diagrammes représentant respectivement, pour deux patients distincts, l'évolution du rapport du coefficient de sensibilité à l'insuline du patient divisé par sa glycémie (en ordonnée, en UI/g/l/g/l), en fonction de la valeur de la variable IPA du patient (en abscisse). Chaque diagramme comprend une pluralité de points 501 correspondant chacun à une mesure du rapport $R^r = CR^r/G^r$ entre le coefficient de sensibilité à l'insuline réel $CR^r$ du patient, et à une valeur correspondante (i.e. corrélée temporellement) de la glycémie réelle $G^r$ du patient.

Le coefficient de sensibilité à l'insuline réel $CR^r$ peut être mesuré par toute méthode connue de mesure du coefficient de sensibilité à l'insuline d'un patient, par exemple par des méthodes du type décrit dans les demandes de brevet US2010/0198520, US2013/0211220 et WO2017/040927.

Dans un mode de réalisation préféré, le coefficient de sensibilité à l'insuline réel $CR^r$ du patient est déterminé à partir de l'historique de glycémie du patient (par exemple mesuré par le capteur 101 dans le système de la figure 1), de son historique de prise de repas, et de son historique d'injection d'insuline, selon la méthode suivante.

A partir de l'historique de données du patient, on identifie des événements de mesure, c'est-à-dire des plages de temps pendant lesquelles le coefficient de sensibilité à l'insuline est isolé, c'est-à-dire pendant lesquelles on observe une diminution de la glycémie du patient liée à l'administration d'insuline. A titre d'exemple, les événements sélectionnés sont des plages temporelles continues allant d'un instant initial $t_{init}$ à un instant final $t_{final}$, répondant aux critères suivants :

- l'instant $t_{init}$ se situe dans une phase d'hyperglycémie, c'est-à-dire une phase dans laquelle la glycémie du patient est supérieure à un seuil prédéterminé, par exemple de l'ordre de 1,40 g/l ;
- un bolus de correction, c'est-à-dire une dose d'insuline supplémentaire a été administré au patient après le début de la phase d'hyperglycémie et avant l'instant $t_{init}$, en vue de limiter la durée de la phase d'hyperglycémie ;
- la glycémie du patient décroit de façon continue entre l'instant initial $t_{init}$ et l'instant final $t_{final}$ ;
- aucune ingestion de glucose par le patient n'a été effectuée entre l'instant $t_{init} - T_j$ et l'instant $t_{final}$, où $T_j$ est une durée de jeun prédéterminée, par exemple supérieure ou égale à 1h et de préférence supérieure ou égale à 2h.

A titre d'exemple, l'instant $t_{init}$ correspond au pic de glycémie de la phase d'hyperglycémie. L'instant $t_{final}$ correspond par exemple à un instant de stabilisation ou de remontée glycémique suivant la phase d'hyperglycémie, ou encore à une perturbation telle qu'un repas ou une ingestion de glucose.

Pour chaque événement identifié, le coefficient de sensibilité à l'insuline réel $CR^r$ du patient est calculé comme suit :

$$CR^r = \Delta I/\Delta G,$$

où $\Delta I$ désigne la quantité d'insuline consommée pendant l'évènement et $\Delta G$ désigne la différence entre la glycémie réelle du patient à l'instant $t_{init}$ de début de l'évènement et la glycémie réelle du patient à l'instant $t_{final}$ de fin de l'évènement. La quantité d'insuline $\Delta I$ consommée pendant l'évènement peut par exemple être calculée en tenant compte des doses d'insuline administrées avant et pendant l'évènement, et de la cinétique d'absorption de l'insuline par le corps. A titre d'exemple, la quantité d'insuline $\Delta I$ consommée pendant l'évènement correspond à la différence entre la quantité d'insuline embarquée ("insulin on board" en anglais) du patient, c'est-à-dire la quantité d'insuline encore active (c'est-à-dire encore susceptible d'avoir un effet sur la glycémie) à l'instant $t_{init}$ de début de l'évènement et la quantité d'insuline embarquée à l'instant $t_{final}$ de fin de l'évènement. La détermination de la quantité d'insuline embarquée du patient aux instants $t_{init}$ et $t_{final}$ peut être réalisée par toute méthode connue de détermination de la quantité d'insuline embarquée d'un patient. A titre d'exemple, la détermination de la quantité d'insuline embarquée du patient à un instant t peut être calculée par convolution, sur une période allant d'un instant antérieur à l'instant t jusqu'à l'instant t, d'une courbe représentative de l'évolution, en fonction du temps, de la quantité d'insuline injectée au patient avant l'instant t, et d'une fonction $f_{IOB}$ représentative de la cinétique de consommation de l'insuline par le corps, par exemple la fonction

$$f_{IOB}(t) = \left(1 + \frac{t-1}{\tau'}\right) \times e^{-\frac{t-1}{\tau'}},$$

où t est la variable temps discrétisée et $\tau'$ est une constante de temps de durée prédéterminée, par exemple comprise entre 40 et 60 minutes, par exemple de l'ordre de 47 minutes.

Pour chaque événement, la valeur de glycémie réelle du patient retenue pour le calcul du ratio $R^r$ est par exemple la valeur de glycémie réelle $G^r(t_{init})$ à l'instant $t_{init}$ de début de l'évènement.

Ainsi, pour chaque événement identifié, le ratio $R^r$ est calculé comme suit : $R^r = CR^r/G^r$ ($t_{init}$).

Pour chaque événement, la valeur retenue du signal IPA est par exemple la valeur IPA($t_{init}$) à l'instant $t_{init}$ de début de l'évènement.

Pour chaque patient, pour définir une fonction ou un modèle mathématique $f_{CR}$ spécifique au patient, un nombre d'évènements $Nb_{ev}$ relativement élevé est d'abord identifié dans les données d'historique du patient, et, pour chaque événement, une valeur du ratio $R^r$ et une valeur du signal IPA associée sont mesurées. A titre d'exemple, le nombre d'évènements $Nb_{ev}$ utilisé pour définir la fonction $f_{CR}$ est compris entre 20 et 100, par exemple entre 30 et 60, par exemple de l'ordre de 40. La fonction $f_{CR}$ est ensuite déterminée par régression à partir des $Nb_{ev}$ points de mesure spécifiques au patient (les points 501 des figures 5A et 5B). A titre d'exemple, pour chaque patient, la fonction $f_{CR}$ est obtenue par régression à partir des $Nb_{ev}$ points de mesure 501 obtenus pour le patient. Pour chaque patient, la fonction $f_{CR}$ est alors une fonction d'équation :

$$f_{CR}(IPA) = a \times IPA^b + c$$

où a, b et c sont des paramètres spécifiques au patient, le paramètre b correspondant à l'ordre du modèle. A titre d'exemple, le paramètre b est fixé égal à 1, le modèle étant alors un modèle linéaire.

Sur chacun des diagrammes des figures 5A et 5B, une droite 503 représente la fonction $f_{CR}$ déterminée pour le patient, reliant la variable IPA du patient au ratio estimé R entre son coefficient de sensibilité à l'insuline et sa glycémie.

A titre d'exemple, dans le système de la figure 1, la fonction $f_{CR}$ peut être déterminée de façon automatisée par l'unité de traitement et de contrôle 105, lors d'une phase de calibration.

L'unité de contrôle et de traitement 105 peut de plus être configurée pour mettre à jour de façon automatisée, par exemple périodiquement, les paramètres de la fonction $f_{CR}$, pour tenir compte des nouvelles données d'historique enregistrées par le système de régulation au fur et à mesure de son utilisation par le patient.

Dans un mode de réalisation préféré, le nombre $Nb_{ev}$ d'évènements pris en compte à chaque mise à jour des paramètres de la fonction $f_{CR}$ reste constant. Autrement dit, à chaque fois qu'un nouvel événement est pris en compte pour la mise à jour des paramètres de la fonction $f_{CR}$, un évènement antérieur, par exemple l'évènement le plus ancien, est exclu du modèle, ce qui permet que le modèle ne se fige pas et puisse évoluer au cours du temps.

La figure 6 est une représentation simplifiée du modèle mathématique $f_{CR}$ utilisé dans le système de la figure 1 pour déterminer le coefficient de sensibilité à l'insuline du patient. Sur la figure 6, le modèle est représenté sous la forme d'un bloc de traitement comportant une entrée e3 sur laquelle est appliquée un signal $G^r(t)$ représentatif de la glycémie réelle du patient, mesurée par le capteur 101, à un instant de mesure t, une entrée e4 sur laquelle est appliqué le signal IPA(t) calculé par l'unité de traitement et de contrôle 105, et une sortie s2 fournissant un signal CR(t) représentatif du coefficient de sensibilité à l'insuline estimé du patient à l'instant t, tel que :

$$CR(t) = G^r(t) \times R(t) = G^r(t) \times f_{CR}(IPA(t))$$

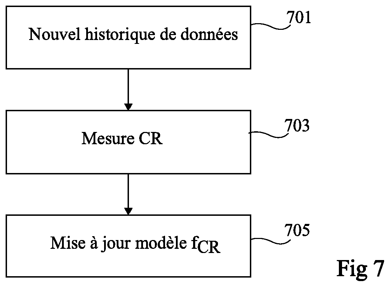

La figure 7 est un diagramme illustrant un exemple d'un procédé pouvant être mis en oeuvre par le système de la figure 1 pour mettre à jour le modèle mathématique $f_{CR}$ pour tenir compte d'un nouvel historique de données du patient.

Le procédé de la figure 7 comprend une étape 701 au cours de laquelle l'unité de traitement et de contrôle acquière et mémorise les données de glycémie réelle $G^r$ mesurée par le capteur 101, les données d'insuline effectivement injectée par le dispositif 103, les données d'ingestion de glucose par le patient, ainsi que les données d'activité physique, sur une période d'observation. La période d'observation considérée à l'étape 701 est choisie pour comprendre au moins un événement permettant une mesure du coefficient de sensibilité réel $CR^r$ du patient.

Le procédé de la figure 7 comprend en outre, après l'étape 701, une étape 703 de calcul du ratio $R^r = CR^r/G^r$ du patient et de la valeur correspondante du signal IPA du patient (par exemple la valeur du signal IPA au début de l'évènement sur lequel est basé le calcul du ratio $R^r = CR^r/G^r$), à partir des données acquises pendant la période d'observation.

Le procédé de la figure 7 comprend de plus, après l'étape 703, une étape 705 de mise à jour du modèle $f_{CR}$ en tenant compte de la nouvelle valeur du ratio $R^r$ et de la valeur correspondante du signal IPA déterminées à l'étape 703.

Des modes de réalisation particuliers ont été décrits. Diverses variantes et modifications apparaîtront à l'homme de l'art.

En particulier, on a décrit ci-dessus des exemples de réalisation dans lesquels un modèle mathématique $f_{CR}$ est utilisé pour calculer, à partir du signal IPA, une première valeur R représentative du ratio $R^r = CR^r/G^r$ du patient, et, par multiplication de cette première valeur avec une valeur de glycémie $G^r$ mesuré par le capteur 101, le coefficient de sensibilité à l'insuline estimé CR du patient (selon la formule susmentionnée $CR(t) = G^r(t) \times R(t) = G^r(t) \times f_{CR}(IPA(t))$).

A titre de variante, le modèle mathématique $f_{CR}$ peut être un modèle à deux variables d'entrée permettant de calculer directement le coefficient estimé CR à partir du signal IPA et d'une valeur de glycémie $G^r$ mesuré par le capteur 101, selon la formule suivante :

$$CR(t) = f_{CR}(IPA(t), G^r(t))$$

Pour chaque patient, la fonction $f_{CR}$ est alors une fonction d'équation :

$$f_{CR}(IPA, G^r) = a \times IPA^b + c \times G^{r^d} + e$$

où a, b, c, d et e sont des paramètres spécifiques au patient, les paramètres b et d correspondant aux ordres du modèle. A titre d'exemple, les paramètres b et d sont fixés égaux à 1, le modèle étant alors un modèle linéaire.

Par ailleurs, les modes de réalisation décrits ne se limitent pas à l'exemple particulier de système de régulation de glycémie décrit en relation avec les figures 1 à 3, à savoir un système à commande prédictive utilisant un modèle mathématique pour prédire l'évolution future de la glycémie du patient et ajuster en conséquence les doses d'insuline à administrer au patient. Plus généralement, la méthode proposée d'estimation en temps réel du coefficient de sensibilité à l'insuline CR du patient, à partir du signal IPA et d'une unique valeur de glycémie mesurée sur le patient, peut être mise en oeuvre dans tout système de régulation de glycémie pouvant tirer profit d'une estimation in-situ et en temps réel du coefficient de sensibilité à l'insuline du patient.

**Revendications**

1. Système automatisé de contrôle de la glycémie d'un patient, comportant un capteur de glycémie (101), un dispositif (107) de mesure d'une activité physique du patient, et une unité de traitement et de contrôle (105), dans lequel :

   l'unité de traitement et de contrôle (105) est configurée pour générer, par convolution d'un signal PA fourni par le dispositif de mesure d'une activité physique du patient avec une fonction mathématique décroissante H, un signal IPA représentatif de l'influence de l'activité physique du patient sur sa sensibilité à l'insuline,
   l'unité de traitement et de contrôle (105) est configurée pour calculer, à partir d'un premier modèle mathématique $f_{CR}$ spécifique au patient et en tenant compte du signal IPA et d'une unique valeur de glycémie $G^r$ mesurée par le capteur (101), un coefficient CR représentatif de la sensibilité à l'insuline du patient, et
   l'unité de traitement et de contrôle (105) est configurée pour mettre en oeuvre une étape de calibration automatique du premier modèle $f_{CR}$ en tenant compte d'un historique de glycémie mesurée par le capteur (101), d'un historique d'insuline injectée au patient, d'un historique d'ingestion de glucose par le patient, et d'un historique du signal d'activité physique PA du patient, sur une période d'observation passée.

2. Système selon la revendication 1, comportant en outre un dispositif d'injection d'insuline (103), dans lequel l'unité de traitement et de contrôle (105) est configurée pour commander le dispositif d'injection d'insuline (103) en tenant compte du coefficient CR.

3. Système selon la revendication 2, dans lequel l'unité de traitement et de contrôle (105) est configurée pour prédire, à partir d'un deuxième modèle mathématique (201), l'évolution future de la glycémie du patient sur une période de prédiction, et à commander le dispositif d'injection d'insuline (103) en tenant compte de cette prédiction.

4. Système selon l'une quelconque des revendications 1 à 3, dans lequel le premier modèle mathématique est une fonction d'équation

$$CR = f_{CR}(IPA, G^r) = a \times IPA^b + c \times G^{r\,d} + e$$

où a, b, c, d et e sont des paramètres spécifiques au patient.

5. Système selon l'une quelconque des revendications 1 à 3, dans lequel le premier modèle mathématique est une fonction d'équation

$$CR = G^r \times f_{CR}(IPA) = G^r \times (a \times IPA^b + c)$$

où a, b et c sont des paramètres spécifiques au patient.

6. Système selon l'une quelconque des revendications 1 à 6, dans lequel l'unité de traitement et de contrôle (105) est configurée pour, lors de l'étape de calibration automatique, mesurer une pluralité de valeurs du coefficient de sensibilité à l'insuline réel $CR^r$ du patient lors d'une pluralité d'événements de mesure compris dans la période d'observation passée.

7. Système selon la revendication 6, dans lequel chaque événement de mesure correspond à une plage temporelle continue allant d'un instant initial $t_{init}$ à un instant final $t_{final}$, répondant aux critères suivants :

   - l'instant $t_{init}$ se situe dans une phase d'hyperglycémie, c'est-à-dire une phase dans laquelle la glycémie du patient est supérieure à un seuil prédéterminé ;

- un bolus de correction, c'est-à-dire une dose d'insuline a été administré au patient après le début de la phase d'hyperglycémie et avant l'instant $t_{init}$, en vue de limiter la durée de la phase d'hyperglycémie ;
- la glycémie du patient décroit de façon continue entre l'instant initial $t_{init}$ et l'instant final $t_{final}$ ; et
- aucune ingestion de glucose par le patient n'a été effectuée entre l'instant $t_{init}$ - $T_j$ et l'instant $t_{final}$, où $T_j$ est une durée de jeun prédéterminée.

8. Système selon la revendication 6 ou 7, dans lequel l'unité de traitement et de contrôle (105) est configurée pour, lors de l'étape de calibration automatique, déterminer le premier modèle mathématique $f_{CR}$ par régression à partir de ladite pluralité de valeurs du coefficient de sensibilité à l'insuline réel $CR^r$.

9. Système selon l'une quelconque des revendications 1 à 8, dans lequel la fonction H est une fonction exponentielle décroissante.

10. Système selon l'une quelconque des revendications 1 à 9, dans lequel le dispositif de mesure (107) comprend une interface utilisateur par laquelle le patient déclare les activités physiques qu'il réalise.

11. Système selon l'une quelconque des revendications 1 à 10, dans lequel le dispositif de mesure (107) comprend un ou plusieurs capteurs adaptés à mesurer des grandeurs représentatives de l'activité physique du patient.

12. Système selon la revendication 11, dans lequel le dispositif de mesure (107) comprend un capteur de mouvement et/ou un capteur de rythme cardiaque.

**Patentansprüche**

1. Ein automatisiertes System zur Kontrolle des Blutglukose bzw. Blutzuckerspiegels eines Patienten, wobei das System Folgendes aufweist:
einen Blutglukose bzw. Blutzuckersensor (101), eine Vorrichtung (107) zum Messen einer körperlichen Aktivität eines Patienten, und eine Verarbeitungs- und Steuereinheit (105), wobei:

die Verarbeitungs- und Steuereinheit (105) konfiguriert ist, ein Signal IPA zu erzeugen, und zwar mittels Konvolution bzw. linearer Überlagerung eines von der Vorrichtung zum Messen einer körperlichen Aktivität des Patienten gelieferten Signals PA mit einer abnehmenden mathematischen Funktion H, wobei das Signal IPA den Einfluss der körperlichen Aktivität des/der Patienten(in) auf seine/ihre Insulinempfindlichkeit darstellt, die Verarbeitungs- und Steuereinheit (105) konfiguriert ist, einen Faktor CR zu berechnen, und zwar aus einem ersten patientenspezifischen mathematischen Modell $f_{CR}$ und unter Berücksichtigung des Signals IPA und aus einem einzelnen vom Sensor (101) gemessenen Blutglukose bzw. Blutzuckerwert $G^r$, wobei der Faktor CR für die Insulinsensitivität des Patienten repräsentativ ist, und die Verarbeitungs- und Steuereinheit (105) konfiguriert ist, einen automatischen Kalibrierungsschritt des ersten Modells $f_{CR}$ auszuführen, und zwar unter Berücksichtigung einer Historie der vom Sensor (101) gemessenen Blutglukose, einer Historie des dem Patienten injizierten Insulins, einer Historie der Kohlenhydrataufnahme durch den Patienten und einer Historie des Signals PA der körperlichen Aktivität des Patienten über einen vergangenen Beobachtungszeitraum.

2. Das System nach Anspruch 1, das ferner eine Insulininjektionsvorrichtung (103) aufweist, wobei die Verarbeitungs- und Steuereinheit (105) konfiguriert ist, die Insulininjektionsvorrichtung (103) unter Berücksichtigung des Faktors CR zu steuern.

3. Das System nach Anspruch 2, wobei die Verarbeitungs- und Steuereinheit (105) konfiguriert ist, anhand eines zweiten mathematischen Modells (201) den zukünftigen Trend der Blutglukose des Patienten über eine Vorhersageperiode vorherzusagen und die Insulininjektionsvorrichtung (103) unter Berücksichtigung der Vorhersage zu steuern.

4. Das System nach einem der Ansprüche 1 bis 3, wobei das erste mathematische Modell eine Funktion der folgenden Gleichung ist:

$$CR = f_{CR}(IPA, G^r) = a \times IPA^b + c \times G^{r\,d} + e$$

wobei a, b, c, d und e patientenspezifische Parameter sind.

5. Das System nach einem der Ansprüche 1 bis 3, wobei das erste mathematische Modell eine Funktion der folgenden Gleichung ist:

$$CR = G^r \times f_{CR}(IPA) = G^r \times (a \times IPA^b + c)$$

wobei a, b und c patientenspezifische Parameter sind.

6. Das System nach einem der Ansprüche 1 bis 5, wobei die Verarbeitungs- und Steuereinheit (105) konfiguriert ist, und zwar während des automatischen Kalibrierungsschritts, eine Vielzahl von Werten des realen Insulinsensitivitätsfaktors $CR^r$ des Patienten während einer Vielzahl von Messereignissen zu messen, die in dem vergangenen Beobachtungszeitraum enthalten sind.

7. Das System nach Anspruch 6, wobei jedes Messereignis einem kontinuierlichen Zeitbereich von einem Anfangszeitpunkt $t_{init}$ bis zu einem Endzeitpunkt $t_{final}$ entspricht, der folgende Kriterien erfüllt:

- Zeitpunkt $t_{init}$ liegt in einer Hyperglykämie-Phase, d.h. in einer Phase, in der die Blutglukose des Patienten über einem vorgegebenen Schwellenwert liegt;
- ein Korrekturbolus, d.h. eine zusätzliche Insulindosis wurde dem Patienten nach Beginn der Hyperglykämie-Phase und vor dem Zeitpunkt $t_{init}$ verabreicht, um die Dauer der Hyperglykämie-Phase zu begrenzen;
- die Blutglukose des Patienten nimmt zwischen dem Anfangszeitpunkt $t_{init}$ und dem Endzeitpunkt $t_{final}$ kontinuierlich ab; und
- zwischen einem Zeitpunkt $t_{init}$ - $T_j$ und dem Zeitpunkt $t_{final}$ hat keine Kohlenhydrataufnahme durch den Patienten stattgefunden, wobei $T_j$ eine vorgegebene Fastendauer ist.

8. Das System nach Anspruch 6 oder 7, wobei die Verarbeitungs- und Steuereinheit (105) konfiguriert ist, während des automatischen Kalibrierungsschritts das erste mathematische Modell $f_{CR}$ mittels Regression aus der Vielzahl von Werten des realen Insulinempfindlichkeitsfaktors $CR^r$ zu bestimmen.

9. Das System nach einem der Ansprüche 1 bis 8, wobei die Funktion H eine abnehmende Exponentialfunktion ist.

10. Das System nach einem der Ansprüche 1 bis 9, wobei die Messvorrichtung (107) eine Benutzerschnittstelle aufweist, über die der/die Patient(in) seine/ihre körperlichen Aktivitäten angibt.

11. Das System nach einem der Ansprüche 1 bis 10, wobei die Messvorrichtung (107) einen oder mehrere Sensoren aufweist, die in der Lage sind, für die körperliche Aktivität des Patienten repräsentative Größen zu messen.

12. Das System nach Anspruch 11, wobei die Messvorrichtung (107) einen Bewegungssensor und/oder einen Herzfrequenzsensor aufweist.

## Claims

1. An automated system for controlling a patient's blood glucose, comprising a blood glucose sensor (101), a device (107) for measuring a physical activity of a patient, and a processing and control unit (105), wherein:

the processing and control unit (105) is configured to generate, by convolution of a signal PA supplied by the device for measuring a physical activity of the patient with a decreasing mathematical function H, a signal IPA representative of the influence of the patient's physical activity on his/her insulin sensitivity,
the processing and control unit (105) is configured to calculate, from a first patient-specific mathematical model $f_{CR}$ and taking into account signal IPA and from a single blood glucose value $G^r$ measured by the sensor (101), a factor CR representative of the patient's insulin sensitivity, and
the processing and control unit (105) is configured to implement a step of automatic calibration of first model $f_{CR}$ by taking into account a history of the blood glucose measured by the sensor (101), a history of insulin injected to the patient, a history of carbohydrate ingestion by the patient, and a history of the patient's physical activity signal PA over a past observation period.

2. The system according to claim 1, further comprising an insulin injection device (103), wherein the processing and control unit (105) is configured to control the insulin injection device (103) by taking into account factor CR.

3. The system according to claim 2, wherein the processing and control unit (105) is configured to predict, from a second mathematical model (201), the future trend of the patient's blood glucose over a prediction period, and to control the insulin injection device (103) by taking the prediction into account.

4. The system according to any of claims 1 to 3, in the first mathematical model is a function of equation

$$CR = f_{CR}(IPA, G^r) = a \times IPA^b + c \times G^{rd} + e$$

where a, b, c, d, and e are patient-specific parameters.

5. The system according to any of claims 1 to 3, wherein the first mathematical model is a function of equation

$$CR = G^r \times f_{CR}(IPA) = G^r \times (a \times IPA^b + c)$$

where a, b, and c are patient-specific parameters.

6. The system according to any of claims 1 to 5, wherein the processing and control unit (105) is configured to, during the automatic calibration step, measure a plurality of values of the patient's real insulin sensitivity factor $CR^r$ during a plurality of measurement events contained in the past observation period.

7. The system according to claim 6, wherein each measurement event corresponds to a continuous time range from an initial time $t_{init}$ to a final time $t_{final}$, complying with the following criteria:

   - time $t_{init}$ is in a hyperglycemia phase, that is, a phase where the patient's blood glucose is greater than a predetermined threshold;
   - a correction bolus, that is, an additional insulin dose has been delivered to the patient after the beginning of the hyperglycemia phase and before time $t_{init}$, to limit the duration of the hyperglycemia phase;
   - the patient's blood glucose continuously decreases between initial time $t_{init}$ and final time $t_{final}$; and
   - no carbohydrate ingestion by the patient has occurred between time $t_{init} - T_j$ and time $t_{final}$, where $T_j$ is a predetermined fasting duration.

8. The system according to claim 6 or 7, wherein the processing and control unit (105) is configured to, during the automatic calibration step, determine the first mathematical model $f_{CR}$ by regression from said plurality of values of the real insulin sensitivity factor $CR^r$.

9. The system of any of claims 1 to 8, wherein function H is a decreasing exponential function.

10. The system according to any of claims 1 to 9, wherein the measurement device (107) comprises a user interface via which the patient declares his/her physical activities.

11. The system according to any of claims 1 to 10, wherein the measurement device (107) comprises one or a plurality of sensors capable of measuring quantities representative of the patient's physical activity.

12. The system according to claim 11, wherein the measurement device (107) comprises a motion sensor and/or a heart rate sensor.

Fig 1

Fig 2

Fig 3

Fig 4A

Fig 4B

Fig 5A

Fig 5B

Fig 6

| Nouvel historique de données | 701 |
| Mesure CR | 703 |
| Mise à jour modèle $f_{CR}$ | 705 |

Fig 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1800493
- FR 1658881
- FR 1658882
- FR 1756960
- US 2010057043 A1
- US 2015217052 A1
- US 20100198520 A
- US 20130211220 A
- WO 2017040927 A

**Littérature non-brevet citée dans la description**

- **ROMAN HOVORKA et al.** Nonlinear model prédictive control of glucose concentration in subjects with type 1 diabètes. *Physiol Meas.,* 2004, vol. 25, 905-920
- **ROMAN HOVORKA et al.** Partitioning glucose distribution/transport, disposal, and endogenous production during IVGTT. *Am J Physiol Endocrinol Metab,* 2002, vol. 282, E992-E1007
- **M. GARNOTEL et al.** Prior automatic posture and activity identification improves physical activity energy expenditure prédiction from hip-worn triaxial accelerometry. *Journal of Applied Physiology,* 1985
- **H. ROMERO-UGALDE et al.** An original piecewise model for computing energy expenditure from accelerometer and heart rate signals. *Physiological measurement,* 28 Juillet 2017, vol. 38 (8), 1599-1615